# EUROPEAN PATENT SPECIFICATION

(11) **EP 3 530 674 B1**
(45) Date of publication and mention of the grant of the patent: **24.04.2024**
(21) Application number: 17861867.4
(22) Date of filing: 19.10.2017
(51) Int. Cl.: C07K 19/00, A61K 39/125, A61P 31/12, C07K 7/08, C07K 14/00, C07K 14/005, C07K 14/08, C12N 15/09, C12P 21/02, C07K 16/10

(54) **POLYPEPTIDE MONOMER, ASSOCIATED PRODUCT FORMED OF SAID POLYPEPTIDE MONOMER HAVING CELL PENETRATION FUNCTION, NOROVIRUS COMPONENT VACCINE FOR SUBCUTANEOUS, INTRADERMAL, PERCUTANEOUS OR INTRAMUSCULAR ADMINISTRATION AND HAVING SAID ASSOCIATED PRODUCT AS EFFECTIVE COMPONENT THEREOF, AND METHOD FOR PRODUCING SAID ASSOCIATED PRODUCT**
POLYPEPTID-MONOMER, DARAUS HERGESTELLTES ZUGEHÖRIGES PRODUKTPOLYPEPTID-MONOMER MIT ZELLPENETRATIONSFUNKTION, NOROVIRUS-KOMPONENTEN-IMPFSTOFF ZUR SUBKUTANEN, INTRADERMALEN, PERKUTANEN ODER INTRAMUSKULÄREN VERABREICHUNG UND MIT BESAGTEMZUGEHÖRIGES PRODUKT ALS WIRKSAME BESTANDTEIL DAVON UND VERFAHREN ZUR HERSTELLUNG DES ZUGEHÖRIGEN PRODUKTS
MONOMÈRE POLYPEPTIDIQUE, PRODUIT ASSOCIÉ FORMÉ DUDITMONOMÈRE POLYPEPTIDE AYANT UNE FONCTION DE PÉNÉTRATION CELLULAIRE, VACCIN COMPOSANT DE NOROVIRUS POUR ADMINISTRATION SOUS-CUTANÉE, INTRADERMIQUE, PERCUTANÉE OU INTRAMUSCULAIRE ET AYANT LEDITPRODUIT ASSOCIÉ EN TANT QUE COMPOSANT EFFICACE DE CELUI-CI, ET PROCÉDÉPOUR LA FABRICATION DUDIT PRODUIT ASSOCIÉ

(30) Priority: 23.10.2016 JP 2016207417
(43) Date of publication of application: 28.08.2019
(73) Proprietor: Denka Company Limited, Tokyo 103-8338 (JP); Tokyo Institute of Technology, Tokyo 152-8550 (JP); The Kitasato Institute, Tokyo 108-8641 (JP); JAPAN AS REPRESENTED BY THE DIRECTOR-GENERAL OF NATIONAL INSTITUTE OF INFECTIOUS DISEASES, Shinjuku-ku Tokyo 162-8640 (JP)
(72) Inventor: UENO Takafumi, Tokyo 152-8550 (JP); YOSHIKAWA Kengo, Tokyo 152-8550 (JP); MANO Megumi, Tokyo 152-8550 (JP); KATAYAMA, Kazuhiko, Tokyo 1088641 (JP); MIKI, Motohiro, Machida-shi Tokyo 1948560 (JP); TODAKA, Reiko, Tokyo 162-8641 (JP); TAKAHASHI, Yoshimasa, Tokyo 162-8640 (JP); ONODERA, Taishi, Tokyo 1628640 (JP)
(74) Representative: Henkel & Partner mbB
(86) International application number: PCT/JP2017/037899
(87) International publication number: WO 2018/074558

(56) References cited:
- WO-A2-2012/140676
- JP-A- 2009 516 529
- JP-A- 2009 542 715
- JP-A- 2015 163 056
- JP-A- 2015 163 056
- JADWIGA CHROBOCZEK ET AL: "Virus-like particles as vaccine", ACTA BIOCHIMICA POLONICA, vol. 61, no. 3, 18 September 2014 (2014-09-18), XP055642717, PL ISSN: 0001-527X, DOI: 10.18388/abp.2014_1875
- T. VESIKARI ET AL: "Norovirus Vaccine: One Step Closer", JOURNAL OF INFECTIOUS DISEASES. JID, vol. 211, no. 6, 9 September 2014 (2014-09-09), pages 853-855, XP055597292, US ISSN: 0022-1899, DOI: 10.1093/infdis/jiu498
- INABA, HIROSHI et al.: "Modulation of cellular functions by protein needles", Biophysics, vol. 55, no. 2, 2015, pages 89-91, XP055477598,
- MAITY, B. et al.: "Design of bioinorganic materials at the interface of coordination and biosupramolecular chemistry", Chem. Rec., vol. 17, no. 4, 1 April 2016 (2016-04-01), pages 383-398, XP055477605, ISSN: 1527-8999, DOI: 10.1002/tcr.201600122
- DATABASE UniProt [Online] 29 April 2015 (2015-04-29), "RecName: Full=Peptidoglycan hydrolase gp36 {ECO:0000305}; EC=3.2.1.17 {ECO:0000312|EMBL:CAD44227.1}; AltName: Full=Gene product 36 {ECO:0000305}; Short=Gp36;", retrieved from EBI accession no. UNIPROT:Q7Y2C9 Database accession no. Q7Y2C9 -& BRIERS Y ET AL: "Stability analysis of the bacteriophage [phi]KMV lysin gp36C and its putative role during infection", CELLULAR AND MOLECULAR LIFE SCIENCES, BIRKHÄUSER-VERLAG, BA, vol. 63, no. 16, 17 July 2006 (2006-07-17) , pages 1899-1905, XP019419227, ISSN: 1420-9071, DOI: 10.1007/S00018-006-6183-7
- Anonymous: "Viral Structural Proteins - MeSH - NCBI", , 1 January 1990 (1990-01-01), XP55779901, Retrieved from the Internet: URL:https://www.ncbi.nlm.nih.gov/mesh?Db=m esh&Cmd=DetailsSearch&Term=%22Viral+Struct ural+Proteins%22%5BMeSH+Terms%5D [retrieved on 2021-02-25]
- Anonymous: "https://www.ncbi.nlm.nih.gov/mesh/?term=P hage+%5BMeSH+Terms%5D", , 1 January 1975 (1975-01-01), XP55779903, Retrieved from the Internet: URL:https://www.ncbi.nlm.nih.gov/mesh/?ter m=Phage+ MeSH+Terms!&report=Full&format=te xt [retrieved on 2021-02-25]

## Description

### Technical Field

The present invention relates to a functional polypeptide, an associated product formed of the polypeptide, a component vaccine containing the polypeptide for subcutaneous, intradermal, percutaneous or intramuscular administration, and a method for producing the associated product. More particularly, the invention relates to an associated product of a polypeptide monomer having a cell penetrating function which monomer is formed of an immunogen and a molecular needle, and to a norovirus component vaccine containing the associated product as an active ingredient (infection protective antigen (hereinafter simply referred to as protective antigen)), for subcutaneous, intradermal, percutaneous or intramuscular administration.

### Background Art

Many vaccines for various infections have been provided, after smallpox vaccine had been developed by Edward Jenner and theoretical support for vaccine had been provided by Louis Pasteur.

Generally, vaccines are divided into three types: a live vaccine, a component vaccine, and a toxoid. The live vaccine employs a pathogenic microorganism (e.g. an attenuated virus) as a protective antigen. The component vaccine employs the entirety or a part of a killed pathogenic microorganism as a protective antigen. The toxoid employs a denatured toxin produced by a pathogen as a protective antigen.

Currently, vaccines are administered to subjects in need thereof mainly via subcutaneous injection or intramuscular injection.

Meanwhile, there has been provided a technique of "molecular needle", which has been developed by focusing on the excellent function of a bacteriophage to introduce genes into a cell (Patent Document 1). More specifically, Patent Document 1 discloses an intracellular introduction agent of a target protein which has low toxicity to a cell, has a high introduction efficiency of a target protein into a cell, and can readily release the target protein from a carrier after intracellular introduction of the target protein. The intracellular introduction agent consists of a polypeptide represented by the general formula Xₙ-Y-Z, wherein X is an amino acid sequence of SEQ ID NO: 1 derived from the β-helix of phage T4, Y is an amino acid sequence selected from SEQ ID NOs: 2 to 5 derived from the C-terminal part of the needle protein of phage T4, P2, Mu or φ92, Z is an amino acid sequence selected from SEQ ID NOs: 6 to 9 derived from the foldon/tip of phage T4, P2, Mu or φ92, and n is an integer of 1 to 8.

Patent Document 2 discloses chimeric polypeptides having antibacterial properties, as well as compositions comprising the same. The chimeric polypeptides comprise (i) a membrane translocation domain (MTD) for traversing the outer membrane of a Gram-negative target bacterium and for penetrating the target bacterium, and (ii) a muralytic domain (MD) having a peptidoglycan degrading enzyme activity for decomposing the cell wall of the target bacterium. In a preferred embodiment, the chimeric polypeptide comprises an amino acid sequence having at least 90% identity to an amino acid sequence SEQ ID NO: 19 of a chimeric polypeptide including the beta helix of gp5 protein from phage T4 as the membrane translocation domain and the catalytic domain of GP36 protein from Pseudomonas phage P134 as the muralytic domain.

Non-Patent Document 1 reviews the use of virus-like particles (VLP) as vaccine, and mentions norovirus VLP vaccines as one example thereof. Non-Patent Document 2 is generically concerned with norovirus vaccines, and describes a combined norovirus VLP-rotavirus VP6 vaccine for protection against childhood gastroenteritis.

### Prior Art Documents

Patent Document 1: JP 2015-163056 A
Patent Document 2: WO 2012/140676 A2
Non-Patent Document 1: Chroboczek et al., "Virus-like particles as vaccine", Acta Biochimica Polonia 61, No. 3 (2014), 531-539
Non-Patent Document 2: Vesikari et al., "Norovirus Vaccine: One Step Closer", Journal of Infectious Diseases 211, No. 6 (2014), 853-855

### Disclosure of the Invention

### Problems to be Solved by the Invention

Application of vaccines to various diseases is promising, but potential problems are involved in use of vaccines.

Vaccines are used to selectively induce immunoreaction by causing simulative infection of target tissue cells with a pathogenic microorganism by use of a protective antigen of interest. A vaccine is administered to blood via subcutaneous injection or intramuscular injection, while the vaccine is expected to be fed at random to target tissue cells by bodily functions. However, at present, it is difficult to efficiently introducing a protective antigen into the target tissue cells. As a result, the protective antigen must be used in an excessive amount greater than the amount of the antigen required for causing immunoreaction.

### Means for Solving the Problems

In order to solve the aforementioned problem, the present inventors have focused on the "molecular needle" as mentioned above. The "molecular needle" has been developed mainly by one of the present inventors.

The inventors have assumed that a molecular needle to which a structural protein of a target virus as an immunogen has been attached, could be introduced into a target tissue cell at high efficiency due to an excellent function of the molecular needle to penetrate cells, via subcutaneous, intradermal, percutaneous or intramuscular administration, to thereby cause an immunoreaction of interest, and the immunogen-attached molecular needle could be used as a protective antigen, and have accomplished the present invention. The invention is defined by the claims.

As an example, when P domain of the capsid protein of a norovirus was used as an immunogen, a hexamer among the associated products falling within the scope of the present invention was found to exhibit excellent viral immunogenicity as a protective antigen. However, biological responses are diverse, and it is not clear at this time what kind of immune phenomenon the associated product of the present invention exhibits when using other immunogens.

The present invention provides the following four aspects:
(1) a "polypeptide" in which an immunogen is attached to a molecular needle (hereinafter also referred to as the polypeptide of the present invention) as defined in claim 1;
(2) an "associated product" formed of the polypeptide as a monomer (hereinafter also referred to as the associated product of the present invention) as defined in claims 3 and 5;
(3) a "norovirus component vaccine for subcutaneous, intradermal, percutaneous or intramuscular administration" as defined in claim 6, the vaccine containing the associated product as an active ingredient (protective antigen) (hereinafter also referred to as the norovirus vaccine of the present invention); and
(4) a method for producing the associated product (hereinafter also referred to as the production method of the present invention) as defined in claim 7.

The "component vaccine" contains an immunization antigen as an essential ingredient. The concept "component vaccine" excludes attenuated live vaccines and toxoids. The immunization antigen employed in the norovirus vaccine of the present invention is the associated product (hexamer) of the present invention serving as a protective antigen.

### (1) Polypeptide of the present invention

The polypeptide of the present invention is a polypeptide represented by the amino acid sequence of the following formula (1). In formulas (1) and (2), the symbol "-" connecting the amino acid sequence units denotes a simple molecular bond (substantially a peptide bond), which is used to clearly distinguish the amino acid sequences each having a specific meaning (e.g. W, L₁, Xₙ and Y) .

Specifically, the polypeptide is represented by

W-L₁-Xₙ-Y ··· (1)

in which W represents an amino acid sequence of the entirety of P domain of the capsid protein of norovirus as an immunogen; L₁ represents a first linker sequence having 10 to 40 amino acids; X represents an amino acid sequence represented by SEQ ID NO: 1; Y represents an amino acid sequence of a cell introduction domain; and repetition number n of X is an integer of 1 to 3,
wherein the amino acid sequence of the cell introduction domain Y is represented by the following formula (2) :

   Y₁-L₂-Y₂-Y₃ ··· (2)
in which Y₁ represents an amino acid sequence of SEQ ID NO: 2; Y₂ represents any one amino acid sequence selected from the group consisting of SEQ ID NOs: 6 to 9; L₂ represents a second linker sequence having 0 to 5 amino acids; Y₃ represents an amino acid sequence for purifying or protecting the polypeptide; and either of Y₂ and Y₃ may be absent.

The number (n) of repetition of amino acid sequence X in Xₙ is preferably 1, but may be 2 or 3.

In the above formula (1), the amino acid sequence represented by Xₙ, Y₁ or Y₂ may be a modified amino acid sequence thereof obtained by deleting, substituting, or adding one or more amino acid residues from, with, or to the original amino acid sequence. The term "deleting" refers to deletion of any amino acid residue in the amino acid sequence included in formula (1) and represented by any of the SEQ ID NOs. The amino acid residue at the N-terminal side of the deleted amino acid residue and that at the C-terminal side of the deleted amino acid residue are linked via a peptide bond. In the case of deletion of the N-terminal amino acid residue or the C-terminal amino acid residue, no linkage is present. The number of deleted residues is counted as "the number of amino acid deletions." The term "substituting" refers to substitution of any amino acid residue in the amino acid sequence included in formula (1) and represented by any of the SEQ ID NOs, "with another amino acid residue." The new amino acid residue is linked to the amino acid residue at the N-terminal side thereof and at the C-terminal side thereof via a peptide bond. In the case of substitution of the N-terminal side or C-terminal side amino acid residue, the amino acid residue is linked via a peptide bond to another C-terminal side or N-terminal side amino acid residue. The number of substituted residues is counted as "the number of amino acid substitutions." The term "adding" refers to addition of one or more new amino acid residues to one or more peptide bond sites in the amino acid sequence included in formula (1) and represented by any of the SEQ ID NOs, to thereby form a new peptide bond(s). The type and number of the above modifications of amino acid residue may be elucidated by comparison in alignment of the amino acid sequence represented by formula (1) with the resultant amino acid sequence through manpower or employment of appropriate analysis software.

The linker sequence L₁ or L₂, or the modification amino acid sequence Y₃ may have any desired sequence within the range of the number of amino acid residues as defined in formula (1).

Further, the hexamer (as described below) of the modified polypeptide having the modified amino acid sequence preferably has substantially the same immunostimulation activity as that of the hexamer of the polypeptide of formula (1). The expression "substantially the same" refers to such a similarity that no significant difference in immunostimulation activity between the hexamer of the polypeptide having the original amino acid sequence and the hexamer of the modified polypeptide having the modified amino acid sequence is confirmed within a significance level range of 5%, when the immunostimulation activity is determined through an established technique such as the "neutralization test."

In the modified amino acid sequence obtained through deletion, substitution, or addition of one or more amino acid residues from, with, or to the amino acid sequence represented by Xₙ, Y₁ or Y₂ included in formula (1), the number of modifications of amino acid residues in each amino acid sequence is ≤ 2n in the case of Xₙ; ≤ 10 in the case of Y₁; and ≤ 5 in the case of Y₂.

### (2) Associated product of the present invention

The associated product of the present invention is formed of the aforementioned polypeptide of the present invention as a monomer. Virtually, the associated product is a trimer (hereinafter also referred to as the trimer of the present invention) or a hexamer (hereinafter also referred to as the hexamer of the present invention). The associated product of the present invention, *per se*, can exert an effect to penetrate cells.

The trimer protein of the present invention is formed of the polypeptide of the present invention as a monomer (protein), wherein the polypeptide monomers may be identical to or different from one another. The hexamer of the present invention is a hexamer protein formed through association of two molecules of the trimer protein.

### (3) The norovirus vaccine of the present invention

The norovirus vaccine of the present invention contains the hexamer of the present invention as an active ingredient (protective antigen). The vaccine is a component vaccine against infection with norovirus and administered in a subcutaneous, intradermal, percutaneous or intramuscular manner. W which is an amino acid sequence of the entirety of P domain of the capsid protein of norovirus as an immunogen.

### (4) The production method of the present invention

The production method of the present invention is a method for producing a polypeptide associated product. In the production method, 3 or more molecules of the polypeptide of the present invention are brought into contact with one another by mediation of an aqueous liquid, to thereby associate the polypeptide molecules as monomers to form a trimer and a hexamer, and the trimer or the hexamer is selectively isolated and recovered.

The polypeptide of the present invention *per se* is formed by genetically expressing a nucleic acid fragment encoding the target polypeptide. Alternatively, the polypeptide is synthesized through a technique of peptide synthesis. When the polypeptide molecules are in contact with one another in an aqueous liquid, a trimer and a hexamer of the polypeptide are spontaneously formed. As a result, a mixture of a trimer and a hexamer of the polypeptide is provided. Through selectively isolating and recovering the trimer or the hexamer, the polypeptide of interest of the present invention (trimer or hexamer) can be yielded.

The "aqueous liquid" will next be described. Particularly when the polypeptide of the present invention is genetically produced, the polypeptide is biologically formed through gene expression. After the production, cells in which the target polypeptide has been formed are collected, and broken or lysed to expose the polypeptide, and the target polypeptide is isolated through a known isolation method. These steps are performed in an aqueous liquid such as water or a buffer. In the aqueous liquid, association proceeds spontaneously, to thereby yield a mixture of the trimer and the hexamer, which are the associated products of the present invention. Alternatively, when the polypeptide of the present invention is produced in whole through chemical synthesis, or produced by synthetizing each divided part and linking them through a chemical modification method, the product is suspended in an aqueous liquid such as water or a buffer. In this case, association spontaneously proceeds, to thereby yield a mixture of the trimer and the hexamer, which are the associated products of the present invention.

No particular limitation is imposed on the method of isolating and recovering the trimer or the hexamer from the mixture, and a known molecular-weight-basis separation method, such as gel electrophoresis, affinity chromatography, molecular sieve separation (e.g., size exclusion chromatography) and ion-exchange chromatography, may be employed.

Thus, one preferred mode of the production method of the present invention is "a method for producing a polypeptide associated product, the method comprising
culturing a transformant into which a nucleic acid fragment encoding the polypeptide of the present invention has been incorporated, in a liquid culture medium, to thereby form the polypeptide through gene expression;
obtaining a mixture of a trimer and a hexamer of the polypeptide (as a monomer), the mixture being formed via a spontaneous association process; and
selectively isolating and recovering the trimer or the hexamer from the mixture."

The thus-produced associated product of the present invention (trimer or hexamer) is purified through a known method, to thereby provide a potentially active ingredient of vaccine. The norovirus vaccine of the present invention is an example of vaccine.

### Effects of the Invention

The present invention can provide the following (1) to (3) :
(1) a polypeptide (monomer) in which a virus structural protein (immunogen) has been attached to a molecular needle, with an expected use as an essential unit of an active ingredient (protective antigen) of a component vaccine for efficiently introducing the virus structural protein (immunogen) into target tissue cells through subcutaneous, intradermal, percutaneous or intramuscular administration;
(2) an associated product which is a trimer or a hexamer of the polypeptide, with an expected use of the active ingredient (protective antigen), and a production method for the associated product; and, as an example of the component vaccines,
(3) a norovirus component vaccine containing the hexamer of the polypeptide to which P domain of the capsid protein of the norovirus serving as an immunogen has been attached, wherein the component vaccine can induce the target immunoreaction through efficiently introducing the hexamer as a protective antigen into the target tissue cells via subcutaneous, intradermal, percutaneous or intramuscular administration.

### Brief Description of the Drawings

[Fig. 1]
   A scheme of forming the associated products of the present invention (trimer and hexamer) from the polypeptide of the present invention.
[Fig. 2]
   A graph showing results of size exclusion column chromatography of "PN-Saga PF," which is an example of the associated product of the present invention.
[Fig. 3]
   A gel filtration chromatographic chart in separation of the associated product of the present invention (hexamer).
[Fig. 4]
   A graph showing test results of rise (in vivo) in antibody titer by subcutaneous administration of the norovirus vaccine of the present invention, indicating the value of the invention.

### Modes for Carrying Out the Invention

### (1) The polypeptide of the present invention

The polypeptide is represented by the following formula (1) :

W-L₁-Xₙ-Y ... (1)

in which W represents an amino acid sequence of the entirety of a virus structural protein as an immunogen; L₁ represents a first linker sequence having 10 to 40 amino acids; X represents an amino acid sequence represented by SEQ ID NO: 1; Y represents an amino acid sequence of a cell introduction domain; and n of X is an integer of 1 to 3.

The immunogen W includes an amino acid sequence based on the entirety of the peptide structure of the virus structural protein. The wording "based on" refers to the immunogen W including not only the original peptide structure but also a modified amino acid sequence such as a detoxicated amino acid sequence obtained by modifying the original amino acid sequence.

The entirety of the virus structural protein is the entirety of a surface portion including the antigen-presenting part, namely the entirety of P domain of the capsid protein of norovirus.

The first linker sequence L₁ is required for appropriately maintaining the distance between the immunogen W and the molecular needle part Y, to thereby suppress steric hindrance. Generally, the number of amino acid residues is preferably larger, as the molecular weight of the immunogen W increases. As described above, the number of amino acid residues is 10 to 40.

X is an amino acid residue represented by SEQ ID NO: 1, and the amino acid sequence Xₙ is a sequence of repeating unit Xs with the number of repetitions n (n is an integer). The mode of repetition is linear repetition. The case of X₂ means "X-X" (wherein symbol "-" denotes a peptide bond). Also, in the repeated sequence Xₙ, the amino acid sequence may be modified in the aforementioned manner. As mentioned above, the number n is an integer of 1 to 3. The number n is preferably 1 but may be 2 or 3. The repeated sequence Xₙ (n = 2 or 3) is employed for consistently maintaining the suitable distance between the molecular needle Y and the immunogen W in response to the dimension and characteristics of the immunogen W.

The cell introduction domain Y is a basement of the molecular needle and is based on the tail needle of a bacteriophage (i.e., cell-penetrating part). The domain Y is a polypeptide represented by the following formula (2):

Y₁-L₂-Y₂-Y₃ ... (2)

in which Y₁ represents an amino acid sequence of SEQ ID NO: 2; Y₂ represents an amino acid sequence selected from the group consisting of SEQ ID NOs: 6 to 9; L₂ represents a second linker sequence having 0 to 5 amino acids; Y₃ represents an amino acid sequence for purifying or protecting the polypeptide; and Y₂ or Y₃ may be absent.

In Y₁ of formula (2), the amino acid sequence from the end of the N-terminal side to the 32 amino acid residue (32 Leu) corresponds to the amino acid sequence of a triple helix β-sheet structure of the bacteriophage T4. The N-terminal amino acid of valine (1 Val) may also be leucine (1 Leu). The remaining C-terminal amino acid sequence is an amino acid sequence of the needle protein of the bacteriophage on the C-terminal side, namely an amino acid sequence (SEQ ID NO: 2) which has the amino acid sequence of gp5 of bacteriophage T4 on the C-terminal side. The nucleotide sequence encoding the amino acid sequence of Y₁ may be selected in accordance with a generally known amino acid-nucleobase relationship.

Y₂ in formula (2) is an amino acid sequence of a domain "foldon" of bacteriophage T4 or an amino acid sequence of a domain "tip" of bacteriophage P2, bacteriophage Mu or bacteriophage φ92. The foldon or the tip is a domain forming the tip portion of the molecular needle structure (i.e. fibritin) of bacteriophage. In formula (2), Y₂ is not necessarily present. However, when it includes the amino acid sequence of the foldon or the tip, the efficiency of incorporation of the molecular needle to the cell membrane can be enhanced. Thus, the presence of Y₂ is preferred. The amino acid sequence of the foldon of bacteriophage T4 is represented by SEQ ID NO: 6. The nucleotide sequence encoding the amino acid sequence may be selected in accordance with a generally known amino acid-nucleobase relationship.

The amino acid sequence of the tip of bacteriophage P2 is represented by SEQ ID NO: 7. The nucleotide sequence encoding the amino acid sequence may be selected in accordance with a generally known amino acid-nucleobase relationship. The amino acid sequence of the tip of bacteriophage Mu is represented by SEQ ID NO: 8. The nucleotide sequence encoding the amino acid sequence may be selected in accordance with a generally known amino acid-nucleobase relationship. The amino acid sequence of the tip of bacteriophage φ92 is represented by SEQ ID NO: 9. The nucleotide sequence encoding the amino acid sequence may be selected in accordance with a generally known amino acid-nucleobase relationship.

L₂ serves as a second linker intervening between Y₁ and Y₂. The number of amino acid residues in linker L₂ is 0 to 5. The number of amino acid residues in linker L₂ of 0 means the absence of second linker L₂.

Y₃ is an amino acid sequence for modification and may be selectively incorporated into Y. The modification amino acid sequence is added in order to purify or protect protein, and examples thereof include tag proteins such as histidine tag, GST tag and FLAG tag. An appropriate linker sequence may be incorporated into Y₃. Such an additional linker sequence may also be a component of the amino acid sequence of Y₃.

The polypeptide of the present invention may be produced through a known method; specifically, a genetic engineering process or chemical synthesis. The entirety of the polypeptide of the present invention may be produced in a single process. Alternatively, segments of the polypeptide are individually produced, and the produced segments are linked through a chemical modification method, to produce the polypeptide. In one linking procedure of polypeptides by mediation of a linker (e.g. L₁ or L₂), a lysine residue or a cysteine residue of one polypeptide may be linked to that of another polypeptide by mediation of a linker having a succinimido group or a maleimido group.

In a genetic engineering process, a nucleic acid fragment encoding the entirety of a part of the target polypeptide of the present invention may be expressed in host cells (e.g. *Escherichia coli*, yeast, insect cells and animal cells) or in a cell-free expression system such as an *E. coli* extract, a rabbit reticulocyte extract or a wheat germ extract. Any nucleic acid expression vector suited for the expression system may be used. Examples of the expression vector include pET (for expression in *E. coli*), pAUR (for expression in yeast), pIEx-1 (for expression in insect cells), pBApo-CMV (for expression in animal cells) and pF3A (for expression in wheat germ extract).

Regarding chemical synthesis, any known method for chemically synthesizing peptides may be employed. More specifically, the entirety or a part of the polypeptide of the present invention may be produced through any established customary method (e.g. liquid phase peptide synthesis or solid phase peptide synthesis). Among generally known suitable solid phase peptide synthesis techniques (i.e. chemical synthesis), a Boc solid phase method or an Fmoc solid phase method may be employed. Also, as described above, a ligation technique may be employed in accordance with need. Needless to say, each amino acid may be produced through a known method, and a commercial product thereof may be used.

### (2) The associated product of the present invention

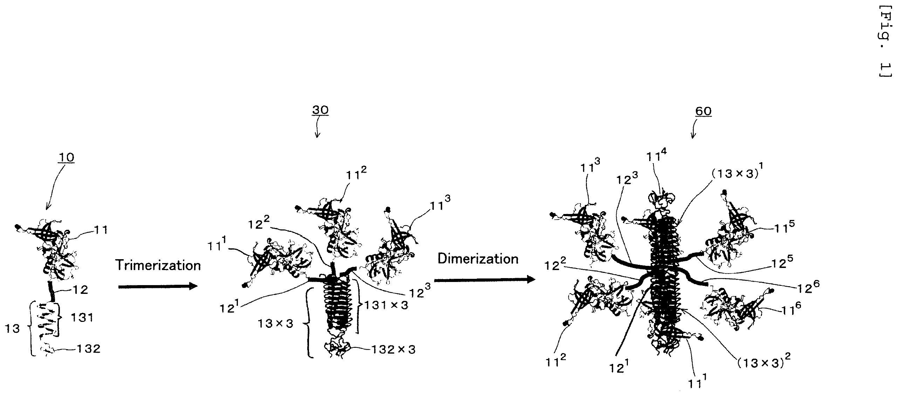

Fig. 1 is a scheme of forming the associated products of the present invention (trimer and hexamer) from the polypeptide of the present invention. In Fig. 1, reference numeral 10 denotes the polypeptide of the present invention in the form of monomer. Reference numeral 30 denotes the trimer of the present invention, and reference numeral 60 denotes the hexamer of the present invention.

The polypeptide 10 of the present invention is formed of a "molecular needle domain 13 corresponding to Xₙ and Y in formula (1)" in which a "base part 131 corresponding to Xₙ in formula (1) and Y₁ in formula (2)" is linked to a "foldon 132 corresponding to Y₂ in formula (2)" and an "immunogen 11 corresponding to W in formula (1)", wherein two components are linked together via a "linker 12 corresponding to L₁ in formula (1)". A linker other than linker 12, and the modification sequence corresponding to Y₃ in formula (2) are not illustrated. The polypeptide 10 of the present invention *per* se substantially exhibits no function of penetrating the cell membrane of the target tissue cells.

The trimer 30 is a spontaneously associated product of three molecules of the polypeptide 10 serving as a monomer via a spontaneous association process. In the trimer 30, 3 units of the molecular needle domain 13 are combined with association via formation of C-terminal-C-terminal bonds, to thereby provide a trimer parallel β-sheet structure, and a helix structure of the β-sheet structure *per se* (i.e. triple helix β-sheet structure), which corresponds to a needle structure. As a result, a molecular needle 13×3 is formed. The molecular needle 13×3 is composed of a basic part 131×3 and a foldon aggregate 132×3. In this way, a "molecular needle" which has a target tissue cell membrane penetration function is formed through trimerization and a self-assembly process. Three linkers (12¹, 12², 12³) originating from the respective monomers, and three immunogen portions (11¹, 11², 11³) linked to the respective linkers exist in the outside area of the molecular needle 13×3.

The hexamer 60 is formed through linking 2 units of the trimer 30, with formation of a bond between the N-terminals of the molecular needle basic parts (13×3)¹ and (13×3)². The hexamer 60 also exhibits a target tissue cell membrane penetration function. Six linkers (12¹, 12², 12³, 12⁵, 12⁶, 12⁴ (not illustrated)) originating from the respective trimers, and six immunogen portions (11¹, 11², 11³, 11⁵, 11⁶, 11⁴ (not illustrated) lined to the respective linkers exist in the outside area of two molecular needles (13×3)¹ and (13×3)².

Trimerization of the polypeptide 10 of the present invention to the trimer 30 and dimerization of the trimer 30 to the hexamer 60 proceed spontaneously in aqueous liquid, and the product exists as the trimer or the hexamer in a stable state. The stability of the trimer or hexamer is remarkably high. For example, the trimer or hexamer is stable in an aqueous liquid at 100°C, in an aqueous liquid at a pH of 2 to 11, or in an aqueous liquid containing 50 to 70 vol.% of organic solvent. In addition, the trimer or hexamer is excellent in safety. When being isolated from the aqueous liquid and dried, the trimer or hexamer is highly stable and retains an excellent cell membrane penetration function.

As described above, transformation of the polypeptide of the present invention to the associated product of interest proceeds spontaneously. Generally, most are the hexamers (final form), but some remains as the trimers.

### (3) The norovirus vaccine of the present invention

The associated product of the present invention (hexamer), serving as an active ingredient of the norovirus vaccine of the present invention, exhibits excellent cell penetration function and immunogenicity. Thus, when administered to target tissue cells via subcutaneous administration, intradermal administration, percutaneous administration or intramuscular administration, the vaccine of the invention efficiently transfers a part or the entirety of P domain of the capsid protein of a norovirus (immunogen) to the target tissue cells, to thereby attain immunization. As a result, the efficacy and safety of the norovirus component vaccine via subcutaneous administration, intradermal administration, percutaneous administration or intramuscular administration are expected to be enhanced.

The norovirus vaccine of the present invention is provided as a pharmaceutical composition (liquid form) for subcutaneous administration, intradermal administration, percutaneous administration or intramuscular administration, which contains, as an active ingredient (protective antigen), a "hexamer including, as W, the P domain of the capsid protein of norovirus", among the associated product as described above. In the case where the associated product of the present invention (hexamer) is directly administered, the associated product is subcutaneously, intradermally, percutaneously or intramuscularly administrated in liquid form which is prepared upon use by suspending and mixing the associated product of the present invention (hexamer) in a buffer or the like. This form is also included in the pharmaceutical composition.

The norovirus vaccine of the present invention may be prepared in a form of a pharmaceutical composition by blending the associated product of the present invention (hexamer) serving as the active ingredient (protective antigen) with an optional adjuvant and an appropriate pharmaceutical carrier. The pharmaceutical carrier may be selected in accordance with the form of use. Examples of the pharmaceutical carrier which may be used in the invention include a filler, an extender, a binder, a humectant, a disintegrant, a surfactant, an excipient and a diluent. As described above, the form of the composition is generally liquid, but may be a dry product, powder, granule and the like which are diluted with liquid upon use.

In the norovirus vaccine of the present invention, the amount of the associated product of the present invention (hexamer) is not necessarily fixed and may be appropriately chosen. Generally, it is preferably in a liquid form which contains 1 to 10 mass% of the associated product of the present invention (hexamer) upon administration. The appropriate single dose of administration (inoculation) is about 0.01 µg to about 10 mg for an adult. As needed, initial inoculation may be appropriately combined with booster inoculation. The administration (inoculation) may be carried out one or more times.

### Examples

The present invention will next be described in detail by way of example.

### [Example 1] Preparation of the associated product of the present invention

### (1) Preparation of the polypeptide of the present invention

In Example 1, four polypeptides having different immunogens (the following (a), (b), (c), and (d)) were prepared through a genetic engineering technique.

(a) MNV-PF:
   a polypeptide employing, as an immunogen, the entirety of one unit of "P domain Full (PF domain)" prepared by deleting N-terminal domain and Shell domain from "VP-1 domain" of a mouse norovirus (hereinafter also referred to as "MNV PF"). The amino acid sequence of the immunogen is represented by SEQ ID NO: 10. The nucleotide sequence encoding the amino acid sequence may be selected in accordance with a generally known amino acid-nucleobase relationship.
(b) MNV-P2:
   a polypeptide employing, as an immunogen, only "P2 domain", among capsid-inside "P1 domain" and capsid-outside "P2 domain" which constitute one unit of "PF domain (P domain Full: PF)" prepared by deleting N-terminal domain and Shell domain from "VP-1 domain" of a mouse norovirus. The amino acid sequence of the immunogen is represented by SEQ ID NO: 11. The nucleotide sequence encoding the amino acid sequence may be selected in accordance with a generally known amino acid-nucleobase relationship.
(c) Saga PF:
   a polypeptide employing, as an immunogen, the entirety of one unit of "P domain" (P domain Full: PF)--one viral capsid antigen of human norovirus. The amino acid sequence of the immunogen is represented by SEQ ID NO: 12. The nucleotide sequence encoding the amino acid sequence may be selected in accordance with a generally known amino acid-nucleobase relationship.
(d) Saga P2:
   a polypeptide employing, as an immunogen, only capsid-outside "P2 domain", among capsid-inside "P1 domain" and capsid-outside "P2 domain which constitute one unit of "P domain" (P domain Full: PF)--one viral capsid antigen of human norovirus. The amino acid sequence of the immunogen is represented by SEQ ID NO: 13. The nucleotide sequence encoding the amino acid sequence may be selected in accordance with a generally known amino acid-nucleobase relationship.

### <Production of plasmid for forming "PN-Saga PF" via expression>

"PN-Saga PF" is a polypeptide represented by the aforementioned formula (1):

W-L₁-Xₙ-Y ··· (1)

in which Y represents a cell introduction domain represented by formula (2):

Y₁-L₂-Y₂-Y₃ ... (2).

In the above formulas, the immunogen W is "Saga PF" represented by amino acid sequence SEQ ID NO: 13; the first linker L₁ is amino acid sequence SEQ ID NO: 15; the repeating unit of the repeating sequence Xₙ is amino acid sequence SEQ ID NO: 1; the number n is 1; the amino acid sequence of the molecular needle base Y₁ is amino acid sequence SEQ ID NO: 2; the second linker L₂ is "SVE"; the amino acid sequence of the foldon Y₂ is amino acid sequence SEQ ID NO: 6; and the amino acid sequence of the modification sequence Y₃ is amino acid sequence SEQ ID NO: 16 (VEHHHHHH).

As mentioned below, expression plasmid "plasmid pKN1-1" for forming a polypeptide in which the immunogen W in the above formulas (1) and (2) is substituted by GFP (green fluorescent protein: SEQ ID NO: 17) was produced. The polypeptide expression plasmid of interest was produced by use of plasmid pKN1-1.

Specifically, a plasmid for forming "PN-Saga PF" was produced through the following procedure.

Firstly, the gene fragment corresponding to 461th to 484th amino acid residues of the wac protein of T4 phage was amplified through PCR from the T4 phage genome, followed by cloning to pUC18, to thereby yield a gene encoding the foldon. Subsequently, the plasmid was cut by restriction enzymes EcoRI and *Sal*I, and the product was inserted into a plasmid pET29b (Novagen) treated by EcoRI and *Xho*I, to thereby yield plasmid pMTf1-3. Also, the gene fragment corresponding to 474th to 575th amino acid residues of gp5 of the T4 phage was amplified through PCR from the T4 phage genome, followed by cloning to pUC18, to thereby yield a gene encoding gp5. Subsequently, the resultant plasmid was cut by restriction enzymes EcoRI and *Sal*I, and the product was inserted into the aforementioned plasmid pMTf1-3 treated by EcoRI and *Xho*I, to thereby yield plasmid pKA176. Separately, the GFP expression vector provided by Takahasi of Gunma University was cut by restriction enzymes *Nde*I and *Eco*RI, to thereby yield a gene encoding the GFP. The product was inserted into the aforementioned plasmid pKA176 treated by restriction enzymes *Nde*I and *Eco*RI, to thereby yield plasmid pKN1-1 (plasmid for GFP-gp5f expression).

On the basis of pKN1-1 serving as the plasmid for GFP-gp5f expression, "plasmid pKN1-SagaPF (or simply referred to as "pKN1-SagaPF") serving as a "PN-Saga PF expression plasmid" was produced by replacing the GFP gene moiety of "plasmid pKN1-1" by Saga PF gene as if cassette exchange was done.

More specifically, a linear plasmid fragment (i.e. receptor plasmid fragment) was produced by removing the GFP gene from plasmid pKN1-1. Specifically, an anti-sense primer (SEQ ID NO: 18) for inverted PCR was synthesized immediately upstream of the GFP gene. Furthermore, a sense primer complimentary to the nucleotide sequence of "MDELYKE" (SEQ ID NO: 14) moiety of the linker sequence L₁ (SEQ ID NO: 15) added to the C-terminal of the GFP protein (SEQ ID NO: 17), was synthesized. Then, by use of the aforementioned antisense primer and the sense primer, inverted PCR was performed with the plasmid pKN1-1 as a template, whereby a linear plasmid fragment free of GFP was yielded as a PCR amplification product (first PCR amplification product).

Subsequently, the Saga PF moiety was amplified, and the product (Saga PF) was incorporated into the above-amplified linear plasmid fragment by use of an In-Fusion cloning kit, as if a cassette was to be inserted. This was carried out through the following PCR process.

The specific procedure is as follows. Firstly, 35-nucleotide sense primer (SEQ ID NO: 20) was synthesized so that the sequence thereof was a 15-nucleotide sense chain nucleotide sequence on the linear vector 5' side and a 20-nucleotide nucleotide sequence on the Saga PF gene sense chain 5' side. In addition, a 35-nucleotide anti-sense primer (SEQ ID NO: 21) was synthesized so that sequence thereof was "a nucleotide sequence of the Saga PF gene 3'-terminal part (corresponding to 7 amino acid residues on the C-terminal amino acid sequence SEQ ID NO: 10 from which the aforementioned linker sequence (SEQ ID NO: 14) had been deleted) and a nucleotide sequence corresponding to MDELY of the linker sequence MDELYKE." Subsequently, the Saga PF fragment was amplified by use of the anti-sense primer and the sense primer and pHuNoV-Saga 1F (human norovirus GII.4 Saga-1 full-length clone) as a template, to thereby yield a PCR amplification product (second PCR amplification product). The sequence on the sense primer side (SEQ ID NO: 22) of the PCR product overlapped with the sequence on the anti-sense primer side of the linear vector PCR product, and the sequence on the anti-sense primer side (SEQ ID NO: 23) of the PCR product overlapped with the sequence on the sense primer side of the linear vector PCR product. The primer which is served as a cassette to be inserted is designed so that the sequence of the primer overlaps with the liner vector sequence at both terminals, and, while the overlapping sequences are employed to maintain the orientation, the primer is joined to the vector by means of the In-Fusion cloning kit.

Finally, the first PCR amplification product and the second PCR amplification product were linked through fusion of respective overlapping sequences by means of the In-Fusion cloning kit, whereby pKN1-Saga PF was produced.

The thus-produced "pKN1-Saga PF" (i.e. a "PN-Saga PF" expression plasmid) was incorporated into *E. coli*, to thereby mass-produce "PN-Saga PF" through expression. The product was purified and employed in the following experiments.

### <Ultracentrifugal analysis of "PN-Saga PF">

The thus-prepared "PN-Saga PF" was subjected to ultracentrifugal analysis. In the specific procedure, 1.2 µM "PN-Saga PF" was subjected to dialysis with 0.1 M sodium phosphate buffer (pH: 7.0), and the product was ultracentrifuged by means of Optimal XL-I analytical ultracentrifuge (Beckman) at 500000 rpm and 20°C.

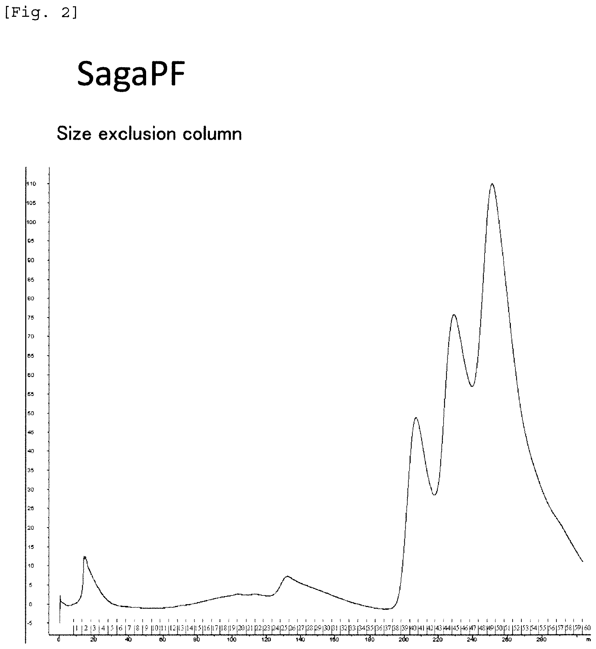

Fig. 2 shows the results of analysis (size exclusion column chromatography) of "PN-Saga PF." As shown in Fig. 2, most of the polypeptides "PN-Saga PF" were found to be spontaneously self-associated, and exist in the form of a "PN-Saga PF" trimer and a hexamer formed from 2 molecules of the trimer.

### [Example 2] Test of introduction of "PN-Saga P2" into cells

The procedure of producing "PN-Saga PF" expression plasmid was repeated, except that the immunogen W was "PN-Saga P2" represented by amino acid sequence SEQ ID NO: 13, to thereby produce a "PN-Saga P2" expression plasmid (pKN1-Saga P2). The formation, purification and ultracentrifugal analysis of "PN-Saga P2" were performed in the same manner as employed in the case of "PN-Saga PF." For replacement of the GFP gene, a sense primer SEQ ID NO: 24 and an anti-sense primer SEQ ID NO: 25 were used for PCR. The thus-obtained PCR product was incorporated into the expression vector PCR product by means of the In-Fusion cloning kit in the same manner as mentioned above.

As a result, most of the polypeptides "PN-Saga P2" were also found to be spontaneously self-associated, and exist in the form of a "PN-Saga P2" trimer and a hexamer formed from 2 molecules of the trimer.

Then, the PN-Saga P2 was added to a supernatant of an HeLa cell culture, so as to investigate the possibility for PN-Saga P2 including the Saga P2 domain to be introduced into target cells. P2 was detected through immunofluorescence using an anit-Saga-1 VLP antibody.

As a result, Saga P2 protein introduced into the HeLa cells was detected as a green fluorescent signal at many spots, whereby it was confirmed that PN-Saga P2 functioned as had been expected (not illustrated, but a photograph can be submitted upon request).

### [Example 3] Studies on "PN-MNV"

The procedure of producing "PN-Saga PF" expression plasmid was repeated, except that the immunogen W was "MNV PF" represented by amino acid sequence SEQ ID NO: 10 or "MNV P2" represented by amino acid sequence SEQ ID NO: 11, to thereby produce a "PN-MNV PF" expression plasmid (pKN1-MNVPF) and a "PN-MNV P2" expression plasmid (pKN1-MNVP2). The procedures of formation, purification, and ultracentrifugal analysis of "PN-MNV PF" and "PN-MNV P2" were performed in the same manner as employed in the case of "PN-Saga PF." For replacement of the GFP gene by the gene of P domain Full, sense primer SEQ ID NO: 26 and anti-sense primer SEQ ID NO: 27 were used in PCR, and for replacement of the GFP gene by the P2 gene of P domain, sense primer SEQ ID NO: 28 and anti-sense primer SEQ ID NO: 29 were used in PCR.

As a result, similar to the case of "PN-Saga PF," most of each of "PN-MNV PF" and "PN-MNV P2" was found to be spontaneously self-associated, and exist in the form of a trimer thereof and a hexamer formed from 2 molecules of the trimer.

Subsequently, introduction of the above "PN-MNV P2" associated product (the associated product of the present invention, also referred to as "PMNVP2 associated product") into HeLa cells as a target was carried out. The results were observed under a confocal microscope.

Firstly, the PMNVP2 associated product was dyed with a fluorescent dye (ATTO520). Specifically, a 7.5 µM suspension of the PMNVP2 associated product in 0.1M sodium phosphate buffer (pH: 8.3) and a 300 µM ATTO solution in DMSO were mixed at a volume ratio of 2 : 1 and at ambient temperature so that the total volume was 450 µL. The dyeing reaction was performed at ambient temperature. After completion of dyeing reaction, the product was washed thrice with 0.1 M sodium phosphate buffer (pH: 7.0) by means of Centricon.

The HeLa cells were preliminarily cultured in 5.0 × 10⁴ cells/100 µL (DMEM) at 37°C under 5% CO₂. Then, the preculture medium was changed to a new medium (total volume: 90 µL) prepared by mixing DMEM (phenol red (-)) and 0.1 M sodium phosphate buffer (pH: 8.3) at a volume ratio of 2 : 1. The ATTO520-added PMNVP2 associated product was added to the new culture system so that the total PMNVP2 concentration was 5 µM. For introducing the PMNVP2 associated product into the HeLa cells, culturing was performed for 30 minutes under the same culture conditions as mentioned above.

Incorporation of the PNMVP2 associated product into HeLa cells was observed under the following conditions using a confocal microscope (laser confocal microscope A1 (Nikon)).
(1) Wavelength of laser light:
   Excitation: 405 nm laser/observation (a 450 nm/50 emission filter) and
   Excitation: 488 nm laser/observation (a 525 nm/50 emission filter)
(2) Laser power
   Laser power: 405 nm: 1.4, 488 nm: 0.4

As a result, the fluorescent dye-added PNMVP2 associated product was observed as a large number of small bright spots around cell nuclei as dark portions. Thus, the PNMVP2 associated product was found to be introduced into HeLa cells for a very short incubation time of 30 minutes (not illustrated, but a photograph can be submitted upon request).

### [Example 4] Mouse immunization test

### (1) Materials

"PN-MNV PF" associated product (PNVPF associated product)
*E. coli* BL21 (DE3), which had been transformed with the "PN-MNV PF" expression plasmid (pKN1-MNVPF) and used in Example 3, was cultured at 37°C for 6 hours in an LB medium (200 mL, kanamycin concentration: 30 µg/mL). Subsequently, an aliquot (30 mL) of the culture liquid was added to a 3L LB medium (kanamycin concentration: 30 µg/mL), and the mixture was cultured at 37°C. When the turbidity (OD 600) reached 0.8 or higher, an IPTG solution was added so as to adjust the final concentration of 1 mM. The resultant mixture was incubated overnight at 20°C. The incubated product was centrifuged at 4°C and 8000 rpm, to thereby collect the bacterial cells. The collected bacterial cells were instantly frozen with liquid nitrogen and stored at -80°C.

One tablet of cOmplete, EDTA-free (Roche) was dissolved in Buffer A (0.1 M Tris-HCl (pH: 8), 0.5 M NaCl, 1 mM DTT, and 5 mM imidazole), and the above bacterial cells (22.4 g) were suspended in the solution. The bacterial cells were broken by ultrasonication. The set of the above operations was performed on ice. Then, the product was centrifuged at 17,500 rpm and 4°C for 50 minutes, and the supernatant was filtered by means of a 0.8 µm filter. The thus-purified supernatant was further purified through Ni affinity column [HisTrap TMHP Colum (GE Healthcare)] at 4°C. Elution was performed with Buffer A and Buffer B (0.1 M Tris-HCl (pH: 8), 0.5 M NaCl, 1 mM DTT, and 500 mM imidazole) under linear gradient conditions (5 to 500 mM imidazole). Samples were monitored at 280 nm, and fractions of interest were recovered.

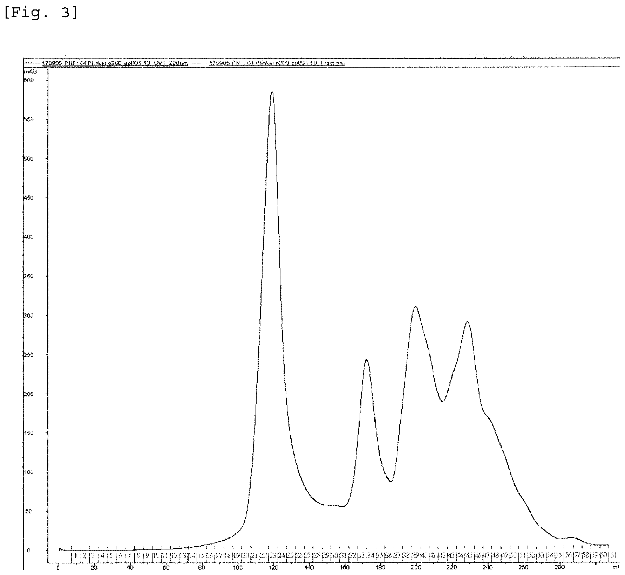

The combined sample obtained through Ni affinity purification was concentrated to a volume of about 10 mL. The product was filtered by means of a 0.2 µm filter. The filtrate was subjected to gel filtration purification by use of HiLoad 26/60 Superdex 200 column (GE Healthcare). The elution was performed by use of Buffer C (20 mM Tris-HCl, 0.5 M NaCl, and 1 mM TCEP). The chart of Fig. 3 shows the results.

The molecular weight of each of the fractions shown in Fig. 3 (each fraction is assigned a fraction number along the horizontal axis) was confirmed through Native SDS PAGE (not illustrated). Among fractions in which a band corresponding to a molecular weight of about 300000 Da (similar to that of the hexamer of interest) was detected, the 36th fraction, which exhibited the sharpest band, was selected. The fraction was employed as a fraction of the PNVPF associated product (hexamer). The trimer thereof may be readily isolated by subjecting the 38th to 40th fractions exhibiting a molecular weight of about 1/2 of the above molecular weight to a customary procedure such as purification through ion chromatography.

The P domain (MNVPF) of the capsid protein of the mouse norovirus was prepared on the basis of a known gene sequence through a customary method as a recombinant protein produced from *E. coli* as a host. More specifically, a sense primer having, on the N-terminal side of the PF domain of the mouse norovirus, an overlapping portion (15 nucleotides) with the upstream side of the cloning site of the expression vector pET6xHN, and an anti-sense primer having, on the C-terminal side, an overlapping portion (15 nucleotides) with the downstream side of the cloning site of the expression vector pET6xHN, were synthesized according to the protocol of In-Fusion cloning kit. Then, PCR was performed by use of the primers, and the thus-obtained PCR product was cloned to pET6xHN by means of the In-Fusion cloning kit, to thereby yield pET6xHN-MNVPF. The pET6xHN-MNVPF was introduced into *E*. *coli* BL21, and MNVPF was expressed through a customary method. The resultant *E. coli* cells were broken, and the entire proteins contained therein were solubilized by use of guanidine. As a result, all the proteins were converted to a linear form. By use of 6 HN tags added to MNVPF contained in the solubilized protein, the product was bound to a column of TALON resin according to the manual thereof. Thereafter, MNVPF was dialyzed with PBS(-), and the solvent was completely changed to PBS(-). After adjustment of the concentration, mice were immunized with the thus-purified MNVPF solution in the following manner.

Besides, a 1 mL syringe with a 26G injection needle (product of TERMO), and mice (Japan SLC, C57BL/6J, 6-week, female) were provided.

### (2) Method

The test systems employing the following two protective antigens were established.
(a) administration of MNVPF (3.12 µg/200 µL PBS)
(b) administration of hexamer (13.2 µg/200 µL PBS)

Each protective antigen diluted solution (total amount: 200 µL) was subcutaneously injected to each mouse at 5 sites (40 µL/site) of the back thereof by means of a syringe with a 26G injection needle. Three weeks after the injection, blood was collected through the tail of the mouse (primarily immune serum). Then, the equal volume of the same antigen diluted solution was subcutaneously injected again to each mouse at the back thereof. Two weeks after the injection, blood was collected through the tail of the mouse (secondarily immune serum).

### <Preparation of serum samples from collected blood>

Each of the thus-collected blood samples was centrifuged by means of a desktop centrifuge at 15000 rpm for 1 minute. The supernatant was transferred to an Eppendorf microtube and centrifuged again at 15000 rpm for 1 minute. The supernatant was transferred to another Eppendorf microtube and employed as a sample for ELISA. ELISA was carried out under the following conditions.

### <Conditions of ELISA>

### (a) Material

·Virus-like hollow particles (VLP) of mouse norovirus
·Mouse serum
·Anti-norovirus monoclonal antibody (G3B9λm IgG 2a, positive control)
·ELISA plate (F96 MAXI SORP NUNC-IMMUNO PLATE, Thermo

### SCIENTIFIC)

·96-well dilution plate (96-well Round Bottom Non-Treated,

### CORNING)

·Tween 20
·BSA
·PBS
·PBST (0.1% Tween 20-added PBS)
·PBSB (1% BSA-added PBS)
·PBSTB (1% BSA, 0.1% Tween 20-added PBS)
·Goat anti-mouse IgG HRP label (Southern Biotech)
·OPD substrate (Sigma, 10 mg tablet of o-phenylenediamine dihydrochloride)
·H₂O₂
·OPD buffer (36 g Na₂HPO₄·12H₂O + 9.6 g citrate (citric acid) + 1 L D₂W, pH: 5.0)
·2N H₂SO₄
·Plate reader (BIORAD iMark)

### (b) Method

A mouse norovirus VLP was mixed with PBS to thereby prepare a 1 µg/mL liquid thereof, and the virus liquid was added to an ELISA plate at 50 µL/well. The ELISA plate was allowed to stand overnight at 4°C and then washed 4 times with PBST. PBST remaining on the plate was completely wiped off, and new PBSB was added to the plate at 80 µL/well. The plate was allowed to stand for 2 hours at room temperature. Separately, a mouse serum was 20-fold diluted with PBSB by use of a dilution plate. The thus-diluted serum was stepwise (3-fold) diluted, to thereby provide 8 dilution series in total. After the above standing for 2 hours, PBSB was removed from the ELISA plate, and the diluted serum was added to the ELISA plate at 50 µL/well. The plate was allowed to stand at room temperature for 4 hours, and the diluted serum was removed from the ELISA plate. The plate was washed 4 times with PBST, and PBST remaining on the plate was completely wiped off. Next, an HRP-labeled anti-mouse IgG antibody (5000-fold diluted with PBSTB) was added to the plate at 50 µL/well, and the plate was allowed to stand for 2 hours at room temperature. Thereafter, the HRP-labeled antibody was removed from the ELISA plate, and the plate was washed 5 times with PBST, followed by completely wiping off remaining PBST. Separately, an OPD substrate (10 mg) was dissolved in an OPD buffer (20 mL), and H₂O₂ (10 µL) was added thereto, followed by inversion mixing. The OPD substrate solution was added to the ELISA plate at 50 µL/well, and the plate was allowed to stand at room temperature for color development. Thereafter, color development was stopped by adding 2N H₂SO₄ to the plate at 50 µL/well. OD490 was measured by means of a plate reader, and the measurements were compared with a positive control to perform concentration calculation.

### (3) Results

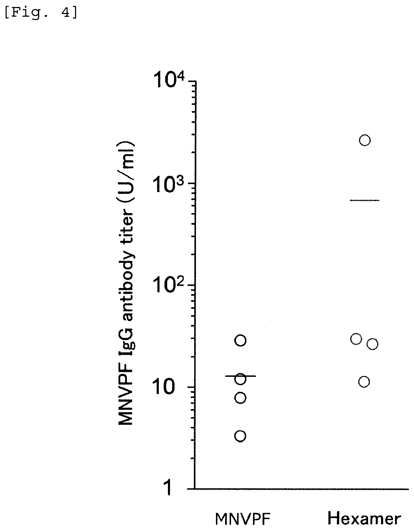

Fig. 4 is a graph showing the results of ELISA after the above initial inoculation. In Fig. 4, the vertical axis represents the IgG antibody titer (U/ml). As shown in Fig. 4, the antibody titer was found to significantly rise in one sample among the 4 samples in the case where the PMNVPF associated product (hexamer) had been inoculated. A tendency of rising in antibody titer was also observed in 2 of the remaining 3 samples. In the case of MNVPF (control), no substantial rise was observed in antibody titer.

That is, the associated product of the present invention (hexamer) having MNVPF as an immunogen was found to be very excellent as a protective antigen.

## Claims

1. A polypeptide represented by the following formula (1):
W-L₁-Xₙ-Y ... (1)
in which W represents an amino acid sequence of the entirety of P domain of the capsid protein of norovirus as an immunogen; L₁ represents a first linker sequence having 10 to 40 amino acids; X represents an amino acid sequence represented by SEQ ID NO: 1; Y represents an amino acid sequence of a cell introduction domain; and repetition number n of X is an integer of 1 to 3,
wherein the amino acid sequence of the cell introduction domain Y is represented by the following formula (2):
Y₁-L₂-Y₂-Y₃ ... (2)
in which Y₁ represents an amino acid sequence of SEQ ID NO: 2; Y₂ represents any one amino acid sequence selected from the group consisting of SEQ ID NOs: 6 to 9; L₂ represents a second linker sequence having 0 to 5 amino acids; Y₃ represents an amino acid sequence for purifying or protecting the polypeptide; and either of Y₂ and Y₃ may be absent,
wherein the amino acid sequence represented by Xₙ, Y₁ or Y₂ may be a modified amino acid sequence thereof obtained by deleting, substituting, or adding one or more amino acid residues from, with, or to the original amino acid sequence represented by Xₙ, Y₁ or Y₂ included in formula (1) or (2), the number of modifications of amino acid residues in each amino acid sequence is ≤ 2n in the case of Xₙ; ≤ 10 in the case of Y₁; and ≤ 5 in the case of Y₂.

2. The polypeptide according to claim 1, wherein the first linker sequence L₁ includes an amino acid sequence represented by SEQ ID NO: 14.

3. A trimer protein formed of a polypeptide as recited in claim 1 or 2 as a monomer protein, wherein the polypeptide monomers are identical to or different from one another.

4. The trimer protein according to claim 3, which includes a parallel β-sheet structure and a helix structure of the parallel β-sheet structure, said parallel β-sheet structure formed by linking Xₙs and Y₁s, respectively, in three molecules of the polypeptide as recited in claim 1 or 2, which molecule are identical to or different from one another.

5. A hexamer protein formed through association of two molecules of a trimer protein as recited in claim 3 or 4.

6. A component vaccine for subcutaneous, intradermal, percutaneous, or intramuscular administration which vaccine contains, as an active ingredient, a hexamer protein as recited in claim 5.

7. A method for producing a polypeptide associated product, which method comprising:
bringing molecules of a polypeptide as recited in any claim 1 or 2 into contact with one another by mediation of an aqueous liquid, to thereby associate the polypeptide molecules to form a trimer and a hexamer; and
selectively isolating and recovering the trimer or the hexamer.

8. The method for producing a polypeptide associated product according to claim 7, wherein the method comprises:
culturing a transformant into which a nucleic acid fragment encoding a polypeptide as recited in claim 1 or 2 has been incorporated, in a liquid culture medium, to thereby form the polypeptide through gene expression;
obtaining a mixture of a trimer and a hexamer of the polypeptide serving as a monomer, the mixture being formed via a spontaneous association process; and
selectively isolating and recovering the trimer or the hexamer from the mixture.

## Patentansprüche

1. Polypeptid wiedergegeben durch die folgende Formel (1):
W-L₁-Xₙ-Y ··· (1)
in der W eine Aminosäuresequenz der Gesamtheit der P-Domäne des Kapsidproteins eines Norovirus als Immunogen ist; L₁ eine erste Linkersequenz mit 10 bis 40 Aminosäuren ist; X eine durch SEQ ID Nr: 1 widergegebene Aminosäuresequenz ist; Y eine Aminosäuresequenz einer Zelleinführungsdomäne ist und die Wiederholungszahl n von X eine ganze Zahl von 1 bis 3 ist,
wobei die Aminosäuresequenz der Zelleinführungsdomäne Y durch die folgende Formel (2) widergegeben wird:
Y₁-L₂-Y₂-Y₃ ··· (2)
in der Y₁ eine Aminosäuresequenz von SEQ ID Nr: 2 ist; Y₂ eine aus der aus SEQ ID Nr.n: 6 bis 9 bestehenden Gruppe ausgewählte Aminosäuresequenz ist; L₂ eine zweite Linkersequenz mit 0 bis 5 Aminosäuren ist; Y₃ eine Aminosäuresequenz zur Reinigung oder zum Schutz des Polypeptids ist und jeder von Y₂ oder Y₃ abwesend sein kann,
wobei die durch Xₙ, Y₁ oder Y₂ wiedergegebene Aminosäuresequenz eine modifizierte Aminosäuresequenz davon sein kann, welche durch Deletieren, durch Substitutieren oder durch Hinzufügen einer oder mehrerer Aminosäurereste von, mit oder zu der ursprünglichen Aminosäuresequenz wiedergegeben durch in der Formel (1) oder (2) enthaltenem Xₙ, Y₁ oder Y₂ erhalten worden ist, wobei die Anzahl der Modifikationen der Aminosäurereste in jeder Aminosäuresequenz in dem Fall von Xₙ ≤ 2; in dem Fall von Y₁ ≤ 10 und in dem Fall von Y₂ ≤ 5 ist.

2. Polypeptid nach Anspruch 1, wobei die erste Linkersequenz L₁ eine durch SEQ ID Nr: 14 wiedergegebene Aminosäuresequenz umfasst.

3. Trimerprotein gebildet aus einem Polypeptid nach Anspruch 1 oder 2 als ein Monomerprotein, wobei die Polypeptidmonomere miteinander identisch oder voneinander verschieden sind.

4. Trimerprotein nach Anspruch 3, welches eine parallele β-Faltblattstruktur und eine Helixstruktur der parallelen β-Faltblattstruktur umfasst, wobei die parallele β-Faltblattstruktur durch die Verknüpfung von X_{nS} bzw. Y_{1S} in drei Molekülen des Polypeptids nach Anspruch 1 oder 2 gebildet worden ist, wobei die Moleküle miteinander identisch oder voneinander verschieden sind.

5. Hexamerprotein, welches durch Assoziation zweier Moleküle eines Trimerproteins nach Anspruch 3 oder 4 gebildet worden ist.

6. Komponentenimpfstoff zur subkutanen, intradermalen, perkutanen oder intramuskulären Verabreichung, wobei der Impfstoff als Wirkstoff ein HexamerProtein nach Anspruch 5 enthält.

7. Verfahren zur Herstellung eines Polypeptid assoziierten Produkts, wobei das Verfahren umfasst:
Inkontaktbringen von Molekülen eines Polypeptids nach Anspruch 1 oder 2 durch Vermittlung einer wässrigen Flüssigkeit, um dadurch die Polypeptidmoleküle zu assoziieren, um dadurch ein Trimer und ein Hexamer zu bilden; und
selektives Isolieren und Gewinnen des Trimers oder des Hexamers.

8. Verfahren zur Herstellung eines Polypeptid assoziierten Produkts nach Anspruch 7, wobei das Verfahren umfasst:
Kultivieren eines Transformanten, in den ein Nukleinsäurefragment, welches ein Polypeptid nach Anspruch 1 oder 2 kodiert, eingebaut worden ist in einem flüssigen Kulturmedium, um dadurch durch Genexpression das Polypeptid zu bilden;
Erhalten einer Mischung aus einem Trimer und einem Hexamer des als Monomer dienenden Polypeptids, wobei die Mischung über einen spontanen Assoziationsprozess gebildet worden ist; und
selektives Isolieren und Gewinnen des Trimers oder des Hexamers aus der Mischung.

## Revendications

1. Polypeptide représenté par la formule (1) suivante :
W-L₁-Xₙ-Y ... (1)
dans lequel W représente une séquence d'acides aminés de l'intégralité du domaine P de la protéine de capside du norovirus en tant qu'immunogène ; L₁ représente une première séquence de liaison ayant 10 à 40 acides aminés ; X représente une séquence d'acides aminés représentée par SEQ ID NO : 1 ; Y représente une séquence d'acides aminés d'un domaine d'introduction cellulaire ; et le nombre de répétitions n de X est un nombre entier de 1 à 3,
dans lequel la séquence d'acides aminés du domaine d'introduction cellulaire Y est représentée par la formule (2) suivante :
Y₁-L₂-Y₂-Y₃ ... (2)
dans lequel Y₁ représente une séquence d'acides aminés de SEQ ID NO : 2 ; Y₂ représente une quelconque séquence d'acides aminés sélectionnée dans le groupe constitué des SEQ ID NO : 6 à 9 ; L₂ représente une deuxième séquence de liaison ayant 0 à 5 acides aminés ; Y₃ représente une séquence d'acides aminés pour purifier ou protéger le polypeptide ; et l'un ou l'autre de Y₂ et Y₃ peut être absent,
dans lequel la séquence d'acides aminés représentée par Xₙ, Y₁ ou Y₂ peut être une séquence d'acides aminés modifiée de celles-ci obtenue par suppression, substitution ou ajout d'un ou de plusieurs résidus d'acide aminé depuis, dans ou à la séquence d'acides aminés d'origine représentée par Xₙ, Y₁ ou Y₂ comprise dans la formule (1) ou (2), le nombre de modifications de résidus d'acide aminé dans chaque séquence d'acides aminés est ≤ 2n dans le cas de Xₙ ; ≤ 10 dans le cas de Y₁ ; et ≤ 5 dans le cas de Y₂.

2. Polypeptide selon la revendication 1, dans lequel la première séquence de liaison L₁ comprend une séquence d'acides aminés représentée par SEQ ID NO : 14.

3. Protéine trimère formée d'un polypeptide selon la revendication 1 ou 2 en tant que protéine monomère, dans laquelle les monomères polypeptidiques sont identiques ou différents les uns des autres.

4. Protéine trimère selon la revendication 3, qui comprend une structure en feuillets β parallèles et une structure en hélice de la structure en feuillets β parallèles, ladite structure en feuillets β parallèles étant formée en liant des Xₙ et des Y₁, respectivement, dans trois molécules du polypeptide selon la revendication 1 ou 2, lesquelles molécules sont identiques ou différentes les unes des autres.

5. Protéine hexamère formée par association de deux molécules d'une protéine trimère selon la revendication 3 ou 4.

6. Vaccin composant pour administration sous-cutanée, intradermique, percutanée ou intramusculaire, lequel vaccin contient, en tant qu'ingrédient actif, une protéine hexamère selon la revendication 5.

7. Procédé de production d'un produit associé à un polypeptide, lequel procédé comprenant :
la mise en contact de molécules d'un polypeptide selon l'une quelconque des revendications 1 ou 2 les unes avec les autres par l'entremise d'un liquide aqueux, pour associer ainsi les molécules de polypeptide pour former un trimère et un hexamère ; et
l'isolement et la récupération sélectifs du trimère ou de l'hexamère.

8. Procédé de production d'un produit associé à un polypeptide selon la revendication 7, dans lequel le procédé comprend :
la culture d'un transformant dans lequel un fragment d'acide nucléique codant pour un polypeptide selon la revendication 1 ou 2 a été incorporé, dans un milieu de culture liquide, pour former ainsi le polypeptide par expression génique ;
l'obtention d'un mélange d'un trimère et d'un hexamère du polypeptide servant de monomère, le mélange étant formé via un processus d'association spontanée ; et
l'isolement et la récupération sélectifs du trimère ou de l'hexamère à partir du mélange.
